(19) Europäisches Patentamt
European Patent Office
Office européen des brevets

(11) Publication number: **0 427 427 A2**

(12) **EUROPEAN PATENT APPLICATION**

(21) Application number: **90311658.0**

(51) Int. Cl.5: **A61K 31/165**, A61K 31/215

(22) Date of filing: **24.10.90**

(30) Priority: **27.10.89 US 427661**

(43) Date of publication of application: **15.05.91 Bulletin 91/20**

(84) Designated Contracting States: **AT BE CH DE DK ES FR GB GR IT LI LU NL SE**

(71) Applicant: **FISONS CORPORATION**
**Jefferson Road P.O. Box 1710**
**Rochester, New York 14603(US)**

(72) Inventor: **Griffith, Ronald Conrad**
**1 Northfield Gate**
**Pittsford, New York 14534(US)**
Inventor: **Napier, James Joseph**
**203 Lake Shore Drive**
**Lindenhurst, Illinois 60046(US)**

(74) Representative: **Wright, Robert Gordon McRae**
**FISONS plc 12 Derby Road**
**Loughborough Leicestershire LE11 0BB(GB)**

# (54) Use of arylalkylamides in the treatment of neurodegenerative diseases.

(57) The use of a compound of Formula I,

$$Ar_1-CH_2-\underset{N-R_2R_3}{\overset{R_1}{C}}-Ar_2 \qquad I$$

or a pharmaceutically acceptable acid addition salt thereof,
wherein $Ar_1$ and $Ar_2$, which may be the same or different, independently represent phenyl or fluorophenyl
$R_1$ represents hydrogen, C1 to 6 alkyl, C1 to 6 alkoxycarbonyl;
$R_2$ represents $COCH_2NH_2$, and
$R_3$ represents hydrogen or C1 to 6 alkyl, as active ingredient in the manufacture of medicament for the treatment and/or prevention of a neurodegenerative disease.

EP 0 427 427 A2

## USE OF ARYLALKYLAMIDES IN THE TREATMENT OF NEURODEGENERATIVE DISEASES

This invention relates to a new use for known pharmaceutical compounds, namely the use of certain arylalkylamides in the treatment and/or prevention of neurodegenerative diseases.

Compounds which possess antihypoxic or N-methyl-(d)-aspartate (NMDA) blocking properties are useful in the treatment and/or prevention of neurodegeneration in pathological conditions such as stroke, cerebral ischaemia, cerebral palsy, hypoglycaemia, Alzheimer's disease, Huntington's chorea, Olivo-pontocerebellar atrophy, perinatal asphyxia and anoxia. Certain arylalkylamides are known from European Patent application 0 287 193 to possess antiepileptic and sedative effects. Surprisingly, we have now found that these compounds possess antihypoxic and/or NMDA blocking properties and are thus useful in the treatment and/or prevention of neurodegenerative diseases.

According to the invention we provide the use of a compound of Formula I,

$$\begin{array}{c} R_1 \\ | \\ Ar_1-CH_2-C-Ar_2 \\ | \\ N-R_2R_3 \end{array} \qquad I$$

or a pharmaceutically acceptable acid addition salt thereof,
wherein $Ar_1$ and $Ar_2$, which may be the same or different, independently represent phenyl or fluorophenyl
$R_1$ represents hydrogen, C1 to 6 alkyl, C1 to 6 alkoxycarbonyl;
$R_2$ represents $COCH_2NH_2$, and
$R_3$ represents hydrogen or C1 to 6 alkyl, as active ingredient in the manufacture of medicament for the treatment and/or prevention of a neurodegenerative disease.

This invention also relates to all stereoisomeric forms and/or optical enantiomeric forms of the compounds of formula I.

The compounds of formula I, and pharmaceutically acceptable salts thereof, are either specifically disclosed in EP -A- 0 279 937 or may be made by methods known per se, including those described in EP-A- 0 279 937.

We prefer compounds of formula I in which one, and more preferably both of $Ar_1$ and $Ar_2$ represent phenyl.

We also prefer those compounds in which $R_1$ represents alkyl, especially methyl.

We further prefer those compounds in which $R_3$ represents hydrogen.

Compounds of formula I which are useful in the methods of treatment or prevention of neurodegenerative disorders include:
2-amino-N-(1,2-diphenyl-1-methylethyl)acetamide
2-amino-N-(1,2-diphenylethyl)acetamide
2-amino-N-[1,2-bis(4-fluorophenyl)-1-methylethyl]acetamide
2-amino-N-(1-ethyl-1,2-diphenylethyl)acetamide
2-amino-N-[1,2-diphenyl-1-(methoxycarbonyl)ethy]acetamide
2-amino-N-methyl-N-(1,2-diphenylethyl)acetamide
(+)-2-amino-N-(1,2-diphenyl-1-methylethyl)acetamide
(-)-2-amino-N-(1,2-diphenyl-1-methylethyl)acetamide
2-amino-N-methyl-N-(1,2-diphenyl-1-methylethyl)acetamide.

Pharmaceutically acceptable acid addition salts of the compounds of formula I include various inorganic or organic salts, such as those formed by reaction of the free base of the compound of formula I with for example, hydrochloric, hydrobromic, sulfuric, phosphoric, acetic, lactic, succinic, fumaric, malic, maleic, tartaric, citric, benzoic, methanesulfonic or carbonic acids.

The compounds of general formula I possess useful pharmaceutical properties. In particular they possess useful Antihypoxia activity and/or NMDA blocking activity for the compounds of formula I, and salts thereof, may be demonstrated in the following screens:

**a) Antihypoxia activity**

Antihypoxia activity, that is, they extension of the lifetime of animals exposed to a hypoxic environment, may be conveniently measured in mice. Groups of mice are tested at various times after the intraperitoneal administration of graded doses of the test compound. The animals' survival time in a temperature controlled hypoxic environment (96% nitrogen and 4% oxygen) is recorded. A statistical comparison is made between coincident vehicle treated animals and the experimental group. The dose-response and minimum active dose (MAD) for compounds are obtained. Other modes of administration can also be used.

**b) NMDA Blocking activity**

NMDA blocking activity may be measured by assessing a compound's ability to protect mice from convulsions induced by intravenous administration of 150 m/k of NMDA according to the procedures of Czuczwar et al., (Neurotransmitters, Seizures and Epilepsy III, edited by G. Nistico et al., Raven Press, New York 1986, pages 235-246). Groups of mice were pretreated by 30 min with the test compound by the oral or intraperitoneal routes and then given NMDA. Animals were observed for convulsions as defined by loss of righting reflex. Animals were kept for 60 min after NMDA dosing and mortality was recorded.

NMDA and glycine receptor affinity may also be tested in the [$^3$H]L-glutamate and [$^3$H]glycine binding assays following the method of Monaghan & Cotman, PNAS, **83**, 7532, (1986) and Watson et al, Neurosci. Res. Comm.,**2**, 169, (1988).

The following **in vitro** methods may also be used to measure NMDA blocking activity: NMDA (20 M) is applied to tissue slices of rat hippocampus (450 m thick) for approximately 2 minutes causing a massive depolarisation of hippocampal neurons, and a profound reduction of the synaptic field potential. The test is repeated seveal times to obtain a base line response. The compound under investigation is then included in the buffer bathing the slice, and NMDA is reapplied. The ability of the compound to block the reduction in field potential produced by NMDA is then determined.

In a second **in vitro** assay, rat hippocampal slices (450 m thick) are pretreated with a buffer containing 10 M 6,7-dinitroquinoxaline-2,3-dione (DNQX) and magnesium (25 M). Under these conditions the synaptic response is almost entirely mediated by NMDA receptors. To evaluate NMDA antagonism, the test compounds are added to the buffer and the synaptic field potentials are compared before and during treatment. The decrease in response caused by the drug is expressed as a percentage of the pre-drug response.

For the above mentioned uses the dosage administered will, of course, vary with the compound employed, the mode of administration and the treatment desired. However, in general, satisfactory results are obtained when the compounds are administered at a daily dosage of from about 0.1 mg to about 20 mg per kg of animal body weight, preferably given in divided doses 1 to 4 times a day or in sustained release form. For man the total daily dose is in the range of from 5 mg to 1,400 mg more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, and pharmaceutically acceptable acid addition salts thereof, may be used on their own or in the form of appropriate medicinal preparations for enteral or parenteral administration.

According to the invention there is also provided a pharmaceutical composition comprising preferably less than 80% and more preferably less than 50% by weight of a compound of formula I, or a pharmaceutically acceptable salt thereof, in admixture with a pharmaceutically acceptable adjuvant, diluent or carrier.

Examples of such adjuvants, diluents and carriers are: for tablets and dragees: lactose, starch, talc, stearic acid; for capsules: tartaric acid or lactose; for injectable solutions: water, alcohols, glycerin, vegetable oils; for suppositories: natural or hardened oils or waxes.

Compositions in a form suitable for oral, i.e. oesophageal administration include tablets, capsules and dragees;

sustained release compositions include those in which the active ingredient is bound to an ion exchange resin which is optionally coated with a diffusion barrier to modify the release properties of the resin.

We prefer the composition to contain up to 50% and more preferably up to 25% by weight of the compound of formula I, or of the pharmaceutically acceptable derivative thereof.

According to a further aspect of the invention, we provide a method of treatment and/or prevention of a neurodegenerative disease, which comprise administration of a therapeutically effective amount of a compound of formula I, or a pharmaceutically acceptable acid addition salt thereof, to a patient suffering from such a disease.

Particular neurodegenrative diseases that may be mentioned include cerebral ischaemia, cerebral palsy,

hypoglycaemia, Alzheimer's disease, Huntington's chorea, Olivo-ponto-cerebellar atrophy, perinatal asphyxia, anoxia and especially stroke.

The compounds of formula I and pharmaceutically acceptable acid addition salts thereof have the advantage that they are less toxic, more efficacious, are longer acting, have a broader range of activity, are more potent, produce fewer side effects, are more easily absorbed or have other useful pharmacological properties, than compounds of similar structure.

The invention is illustrated by the following examples, in which compound A is 2-amino-N-(1,2-diphenyl-1-methylethyl)acetamide and compound B is 2-amino-N-(1,2-diphenylethyl) acetamide:

| Example 1 | | | |
|---|---|---|---|
| Mice - Antihypoxia studies | | | |
| compound | oral ED50 | oral TD50 | TI(TD50/ED50) |
| A<br>B | 55.5<br>19.1 | 876<br>236 | 15.8<br>12.4 |
| The ED50 measures the dose that produces a 50% increase in the time to mortality when mice are exposed to an hypoxic environment. The TD50 test is the inverted screen test of Coughenour et al., Pharm. Biochem. Behav. 6: p351, 1977. | | | |

| Example 2 | | | |
|---|---|---|---|
| Mice - Protection Against NMDLA (N-methyl-D,L-aspartate) induced seizures and subsequent mortality. | | | |
| compound | ip ED50 | ip TD50 | TI(TD50/ED50) |
| NMDLA convulsions | | | |
| A<br>B | 57.4<br>33 | 85.2<br>82.4* | 1.5<br>2.5 |
| NMDLA mortality | | | |
| A<br>B | 22.4<br>20.6 | 85.2<br>82.4* | 3.8<br>4.0 |
| Protection against convulsions is evaluated for 5 minutes following iv injection of 150 mg/kg of NMLDA, while protection against mortality is evaluated over a period of 60 minutes after NMLDA injection. | | | |

* No TD50 on the inverted screen was performed. This concentration represents a CD50 value (the dose when 50% of mice experience a seizure following ip injection).

Example 3

Rats - Rats are subjected to the 4-vessel occlusion model of stroke for 30 minutes followed by drug

injection at 30 minutes after blood reflow to the brain. The drugs are administered ip twice daily for one week. At 8 days post stroke the brains are removed and protection is determined by determining viability of the vulnerable CA1 neurons in the hippocampus (neutrons associated with short term memory). Both Compound A and Compound B were effective in preventing loss of the CA1 neurons at the ip doses of 20mg/kg. The ip doses for TD50s relative to neurotoxicity in the rat are 76 for Compound A and 47 for Compound B. Therefore one could calculate a therapeutic range of 3.8 for Compound A and a value of 2.4 for Compound B. The TD50 in rats is determined by administering the compound and one hour later the rats are required to walk a narrow plank to escape into a darkened box. Rats that fall are scored as failures.

Example 4

Dogs - Dogs are subjected to 8 minutes global ischemia (clamping the ascending aorta) and are treated iv with drug at 30 minutes after reflow. Treatment is given 6 hours later, b.i.d. for 2 more days and s.i.d. for 4 additional days. On day 8 the brains are removed and again drug protection of the vulnerable CA1 neurons is assessed. The Toxic doses are determined by administering the drug iv to conscious dogs in incremental doses and observing for side effects (we use preictal behaviour or convulsions as an endpoint). Compound A was active in protecting dogs against stroke at 7.5mg/kg while Compound B was active at 5mg/kg. In the toxicity studies the lowest toxic dose of Compound A was 14mg/kg for preictal behaviour and 44mg/kg for convulsions giving therapeutic ranges of around 1.9 (preictal behavioural index) or 5.9 (convulsive index). The toxicity of Compound B for both preictal behaviour and convulsions in dogs was observed at 44mg/kg which would give a therapeutic range of about 8.8 for this compound.

## Claims

1. The use of a compound of Formula I,

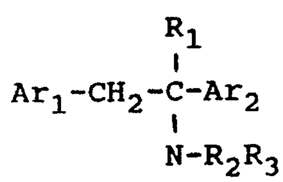

I

or a pharmaceutically acceptable acid addition salt thereof,
wherein $Ar_1$ and $Ar_2$, which may be the same or different, independently represent phenyl or fluorophenyl
$R_1$ represents hydrogen, C1 to 6 alkyl, C1 to 6 alkoxycarbonyl ;
$R_2$ represents $COCH_2NH_2$, and
$R_3$ represents hydrogen or C1 to 6 alkyl, as active ingredient, in the manufacture of medicament for the treatment and/or prevention of a neurodegenerative disease.

2. A use according to Claim 1, wherein at least one of $Ar_1$ and $Ar_2$ represents phenyl.

3. A use according to Claim 1 or Claim 2, wherein $R_1$ represents C1 to 6 alkyl.

4. A use according to any one of Claims 1, 2 or 3, wherein $R_3$ represents hydrogen.

5. A use according to any one of the preceding Claims, wherein the compound of formula I is
2-amino-N-(1,2-diphenyl-1-methylethyl)acetamide, or a pharmaceutically acceptable acid addition salt thereof.

6. A use according to Claim 1, wherein the compound of formula I is
2-amino-N-(1,2-diphenylethyl)acetamide
2-amino-N-[1,2-bis(4-fluorophenyl)-1-methylethyl]acetamide
2-amino-N-(1-ethyl-1,2-diphenylethyl)acetamide
2-amino-N-[1,2-diphenyl-1-(methoxycarbonyl)ethy]acetamide
2-amino-N-methyl-N-(1,2-diphenylethyl)acetamide
2-amino-N-methyl-N-(1,2-diphenyl-1-methylethyl)acetamide, or a pharmaceutically acceptable acid addition salt thereof.

7. A use according to any of the preceding Claims wherein the neurodegenerative disease is selected from cerebral ischaemia, cerebral palsy, hypoglycaemia, Alzheimer's disease, Huntington's chorea, Olivo-ponto-cerebellar atrophy, perinatal asphyxia, anoxia and stroke.

8. A use according to any of the preceding Claims, wherein the neurodegenerative disease is stroke.

Claims for the following Contracting States: ES, GR

1. The use of a compound of Formula I,

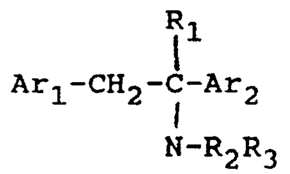

I

or a pharmaceutically acceptable acid addition salt thereof,
wherein $Ar_1$ and $Ar_2$, which may be the same or different, independently represent phenyl or fluorophenyl
$R_1$ represents hydrogen, C1 to 6 alkyl, C1 to 6 alkoxycarbonyl;
$R_2$ represents $COCH_2NH_2$, and
$R_3$ represents hydrogen or C1 to 6 alkyl, as active ingredient, in the manufacture of medicament for the treatment and/or prevention of a neurodegenerative disease.

2. A use according to Claim 1, wherein at least one of $Ar_1$ and $Ar_2$ represents phenyl.

3. A use according to Claim 1 or Claim 2, wherein $R_1$ represents C1 to 6 alkyl.

4. A use according to any one of Claims 1, 2 or 3, wherein $R_3$ represents hydrogen.

5. A use according to any one of the preceding Claims, wherein the compound of formula I is
2-amino-N-(1,2-diphenyl-1-methylethyl)acetamide, or a pharmaceutically acceptable acid addition salt thereof.

6. A use according to Claim 1, wherein the compound of formula I is
2-amino-N-(1,2-diphenylethyl)acetamide
2-amino-N-[1,2-bis(4-fluorophenyl)-1-methylethyl]acetamide
2-amino-N-(1-ethyl-1,2-diphenylethyl)acetamide
2-amino-N-(1,2-diphenyl-1-(methoxycarbonyl)ethy]acetamide
2-amino-N-methyl-N-(1,2-diphenylethyl)acetamide
2-amino-N-methyl-N-(1,2-diphenyl-1-methylethyl)acetamide, or a pharmaceutically acceptable acid addition salt thereof.

7. A use according to any of the preceding Claims wherein the neurodegenerative disease is selected from cerebral ischaemia, cerebral palsy, hypoglycaemia, Alzheimer's disease, Huntington's chorea, Olivo-ponto-cerebellar atrophy, perinatal asphyxia, anoxia and stroke.

8. A use according to any of the preceding Claims, wherein the neurodegenerative disease is stroke.

9. A process for the preparation of a medicament for use in the treatment or prevention of a neurodegenerative disease, which comprises mixing a compound of formula I as defined in Claim 1, or a pharmaceutically acceptable acid addition salt thereof, with a pharmaceutically acceptable adjuvant, diluent or excipient.